# EUROPEAN PATENT SPECIFICATION

(11) **EP 3 525 783 B1**
(45) Date of publication and mention of the grant of the patent: **16.12.2020**
(21) Application number: 17791004.9
(22) Date of filing: 13.10.2017
(51) Int. Cl.: A61K 31/409, A61K 31/7068, A61K 33/24, A61K 41/00, A61P 35/00

(54) **TREATMENT OF CHOLANGIOCARCINOMA WITH TPCS-2A INDUCED PHOTOCHEMICAL INTERNALISATION OF GEMCITABINE**
BEHANDLUNG DES CHOLANGIOKARZINOMS MIT TPCS-2A-INDUZIERTER PHOTOCHEMISCHER INTERNALISIERUNG VON GEMCITABIN
TRAITEMENT DE CHOLANGIOCARCINOME AVEC INTERNALISATION PHOTOCHIMIQUE DE GEMCITABINE INDUITE PAR TPCS-2A

(30) Priority: 14.10.2016 GB 201617526; 24.03.2017 GB 201704719
(43) Date of publication of application: 21.08.2019
(73) Proprietor: PCI Biotech AS, 0379 Oslo (NO)
(72) Inventor: HØGSET, Anders, 0681 Oslo (NO); WALDAY, Per Edvard, 0273 Oslo (NO); SELBO, Pål Kristian, 0373 Oslo (NO); EIVINDVIK, Kristin, 1364 Fornebu (NO); FINNESAND, Lena, 0491 Oslo (NO)
(74) Representative: Dehns
(86) International application number: PCT/EP2017/076257
(87) International publication number: WO 2018/069536

(56) References cited:
- Anonymous: "A Phase I/II Study Safety and Efficacy Study of PCI of Gemcitabine and Chemotherapy in Patients With Cholangiocarcinomas NCT01900158", ClinicalTrials.gov, 13 October 2016 (2016-10-13), pages 1-7, XP055431824, Retrieved from the Internet: URL:https://www.clinicaltrials.gov/ct2/sho w/record/NCT01900158?term=amphinex&cond=ch olangiocarcinoma&rank=1 [retrieved on 2017-12-05]
- Anonymous: "Positive results on bile duct cancer from PCI Biotech", Oslo Cancer Cluster, 24 April 2014 (2014-04-24), pages 1-3, XP055431839, Retrieved from the Internet: URL:http://oslocancercluster.no/2014/04/24 /positive-results-on-bile-duct-cancer-from -pci-biotech/ [retrieved on 2017-12-05]
- ALEJANDRA MARTINEZ DE PINILLOS BAYONA ET AL: "Enhancing the efficacy of cytotoxic agents for cancer therapy using photochemical internalisation", INTERNATIONAL JOURNAL OF CANCER, vol. 138, no. 5, 23 March 2015 (2015-03-23), pages 1049-1057, XP055431897, US ISSN: 0020-7136, DOI: 10.1002/ijc.29510
- Richard Sturgess ET AL: "PHASE I STUDY WITH PHOTOCHEMICAL INTERNALISATION (PCI)- A NOVEL TECHNOLOGY FOR TREATMENT OF PERIHILAR CHOLANGIOCARCINOMA 1", , 14 October 2016 (2016-10-14), pages 1-11, XP055431840, Retrieved from the Internet: URL:http://ammf.org.uk/wp-content/uploads/ 2016/10/Phase-I-CCA-UEGW-October-2016.pdf [retrieved on 2017-12-05]
- STURGESS R ET AL: "Phase I clinical study with photochemical internalisation, a novel technology for treatment of perihilar cholangiocarcinoma", JOURNAL OF HEPATOLOGY, vol. 66, no. 1, 21 April 2017 (2017-04-21) , XP085012364, ISSN: 0168-8278, DOI: 10.1016/S0168-8278(17)31282-5
- Anonymous: "A trial looking at photochemical internalisation (PCI) for advanced bile duct cancer", , 20 October 2016 (2016-10-20), pages 1-6, XP055432727, Retrieved from the Internet: URL:http://www.cancerresearchuk.org/about- cancer/find-a-clinical-trial/a-trial-looki ng-at-photochemical-internalisation-pci-fo r-advanced-bile-duct-cancer#undefined [retrieved on 2017-12-07]

## Description

The present invention concerns gemcitabine and TPCS₂ₐ and optionally another cytotoxic agent, preferably cisplatin, for use in treating a cholangiocarcinoma in a patient comprising the systemic administration of TPCS₂ₐ to the patient and subsequently systemically administering gemcitabine and irradiating the cholangiocarcinoma via a suitably placed optical fiber, as defined in the claims. This treatment may be coupled with further systemic administration of gemcitabine and/or another cytotoxic agent, preferably cisplatin.

Bile duct cancer (also referred to as cholangiocarcinoma, CCA) is a rare but usually fatal disease. CCAs are divided into intrahepatic and extrahepatic CCAs, the latter which may be sub-divided into perihilar and distal CCAs. Over 90% of CCAs are adenocarcinomas. CCA has an incidence of approximately 1.7/100,000 in the population. A higher prevalence is found in Asia, in particular in China. The number of new cases in the USA and EU alone is approximately 11,000 per year. 60 to 80% of CCAs are extrahepatic and 70-80% of these are not candidates for curative resection. The five year survival rate is less than 5% and 0% when the tumour is inoperable. The average survival for inoperable CCAs is 12 months.

Currently CCAs are managed by surgery, stenting and/or chemotherapy. Surgery is the only potentially curative treatment for CCA. However, less than a third of the tumours are resectable at presentation. Endoscopic stenting is the procedure of choice for palliative biliary drainage in patients with unresectable disease. There is no approved chemotherapy for CCA treatment. Recommended chemotherapy treatment includes a combination of gemcitabine and cisplatin but is not curative. Valle et al., 2010, New England J. Med., 362, pp 1273-1281 report experiments in which cisplatin and gemcitabine were administered to patients with locally advanced or metastatic biliary tract cancer and achieved median overall survival of 11.7 months.

There remains a great need for a treatment for CCAs, particularly for those which are inoperable and for which no curative treatments exist.

Photochemical internalisation (PCI) is a known technique which improves delivery of molecules into the cytosol. This may be used to internalize molecules to affect the function of the cells (e.g. cytotoxic molecules to kill the cells) or to allow their presentation on the cell surface, e.g. in methods of vaccination. PCI is a technique which uses a photosensitizing agent, in combination with an irradiation step to activate that agent, and is known to achieve release of molecules co-administered to a cell into the cell's cytosol. This technique allows molecules that are taken up by the cell into organelles, such as endosomes, to be released from these organelles into the cytosol, following irradiation. PCI provides a mechanism for introducing otherwise membrane-impermeable (or poorly permeable) molecules into the cytosol of a cell in a manner which does not result in widespread cell destruction or cell death.

The basic method of photochemical internalisation (PCI), is described in WO 96/07432 and WO 00/54802. In such methods, the molecule to be internalised (which in the present invention would be the cytotoxic agents), and a photosensitizing agent are brought into contact with a cell. The photosensitizing agent and the molecule to be internalised are taken up into a cellular membrane-bound subcompartment within the cell, i.e. they are endocytosed into an intracellular vesicle (e.g. a lysosome or endosome). On exposure of the cell to light of the appropriate wavelength, the photosensitizing agent is activated which directly or indirectly generates reactive species which disrupt the intracellular vesicle's membranes. This allows the internalized molecule to be released into the cytosol.

It was found that in such a method the functionality or the viability of the majority of the cells was not deleteriously affected. Thus, the utility of such a method, termed "photochemical internalisation" was proposed for transporting a variety of different molecules, including therapeutic agents, into the cytosol i.e. into the interior of a cell.

PCI has been shown to enhance biological activity of a large variety of macromolecules and other molecules that do not readily penetrate through plasma membrane including type-I ribosome-inactivating proteins, immunotoxins, chemotherapeutic agents such as Bleomycin (Blenoxane®) and Doxorubicin, gene encoding plasmids and oligonucleotides. It has been found to induce cytotoxicity in deeper tissue layers than the corresponding photodynamic therapy (PDT). Due to the combination of targeted therapeutics with light-activated cytosolic delivery induced by photosensitisers preferentially accumulating in solid tumors, PCI can be highly specific and this also contributes to enhanced antitumor efficacy. NCT01900158 (ClinicalTrials.gov) discloses a Phase I/II safety and efficacy study of PCI of gemcitabine and chemotherapy in patients with cholangiocarcinomas.

As noted above, CCAs are in need of a medical treatment that can provide alternatives to existing methods which, at present, offer limited improvements to life expectancy for patients. The present invention addresses this need.

The present inventors have surprisingly found that, advantageously, a method involving the use of a photosensitizing agent, TPCS₂ₐ, and gemcitabine at the doses defined herein, and irradiation with light of a wavelength effective to activate the photosensitizing agent results in significant improvements relative to standard treatments. As will be described in more detail in the Examples below, it has been demonstrated that after 6 months of treatment more than 80% of lesions had shrunk and more than 50% of lesions were no longer detectable. This is a remarkable and important result which offers new hope for the treatment of CCAs. The result is particularly surprising as the PCI method relies on release of molecules in the endosome into the cytosol and there was nothing to suggest that gemcitabine was taken up in cells into the endosome and hence could benefit from PCI treatment.

The invention is defined by the claims. Any subject-matter falling outside the scope of the claims is provided for information purposes only. Thus, in a first aspect the present invention provides gemcitabine and TPCS₂ₐ and optionally another cytotoxic agent, preferably cisplatin, for use in treating a cholangiocarcinoma in a human patient, wherein i) said TPCS₂ₐ is to be systemically administered to said patient to a dose of 0.05 to 0.5 mg/kg; and ii) after 3-5 days, said gemcitabine is to be systemically administered to said patient to a dose of 500-1500 mg/m² and said cholangiocarcinoma is to be irradiated with light with a wavelength of 640-665nm using an optical fiber placed within 3cm of said cholangiocarcinoma to provide a light dose of 10 to 60 J/cm; and optionally iii) after 1-40 days (preferably after 7-21 days) gemcitabine and/or another cytotoxic agent, preferably cisplatin, is to be systemically administered to said patient.

As defined herein "treating" refers to reducing, alleviating or eliminating one or more symptoms of the CCA which is being treated, relative to the symptoms prior to treatment. In particular said treatment may comprise reduction in the size or volume of the CCA being treated. The treatment may include effects on the CCA closest to the optical fiber as well as other CCAs which are more distant and may not directly receive irradiation from the light source. In one aspect of the invention the method treats one or more cholangiocarcinoma (or cells therefore) which are not irradiated during the irradiation step (this includes direct or indirect irradiation, i.e. which do not receive a light dose resulting from the irradiation step).

A "cholangiocarcinoma" is a bile duct cancer which may be intrahepatic or extrahepatic (which may be perihilar and distal). Over 90% of CCAs are adenocarcinomas.

The "patient" is a human.

"Systemic administration" includes any form of non-local administration in which the agent is administered to the body at a site other than directly adjacent to, or in the local vicinity of, the CCA, resulting in the whole body receiving the administered agent. Conveniently, systemic administration may be via enteral delivery (e.g. oral) or parenteral delivery (e.g. intravenous, intramuscular or subcutaneous). Intravenous delivery is preferred.

"TPCS₂ₐ" is a photosensitizing agent (tetraphenyl chlorin disulphonic acid) having the structure below or isomers thereof. The structure below provides the 7,8-dihydro isomer. The 12,13- and 17,18-dihydro isomers are encompassed by the term TPCS₂ₐ. Pharmaceutically acceptable salts thereof are also encompassed. Such molecules are as described in WO03/020309 and WO2011/018635.

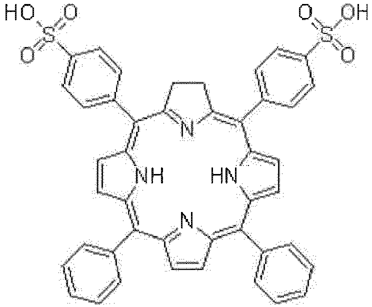

TPCS₂ₐ may be obtained from BOC Sciences, NY, USA or from PCI Biotech AS, Norway. Alternatively, TPCS₂ₐ may be prepared as described in WO03/020309 and WO2011/018635 . Pharmaceutically acceptable salts include acid addition salts with physiologically acceptable organic or inorganic acids. Suitable acids include, for example, hydrochloric, hydrobromic, sulphuric, phosphoric, acetic, lactic, citric, tartaric, succinic, maleic, fumaric and ascorbic acids. Hydrophobic salt may also conveniently be produced by, for example, precipitation. Appropriate salts include, for example, acetate, bromide, chloride, citrate, hydrochloride, maleate, mesylate, nitrate, phosphate, sulphate, tartrate, oleate, stearate, tosylate, calcium, meglumine, potassium and sodium salts. Procedures for salt formation are conventional in the art.

Preferred salts include diethanolamine salt, ethanolamine salt (preferably bis (monoethanolamine)), N-methyl-glucamine salt, triethanolamine salt, 1-(2-hydroxymethyl)-pyrrolidine salt and 2-amino-2-(hydroxymethyl) propane-1,3-diol salt.

"Pharmaceutically acceptable" as referred to herein refers to ingredients that are compatible with other ingredients used in the methods or uses of the invention as well as physiologically acceptable to the recipient.

The agents used herein, e.g. the TPCS₂ₐ, gemcitabine and/or other cytotoxic agent may be provided in the form of a pharmaceutical composition for use in the method of the invention and may be formulated in any convenient manner according to techniques and procedures known in the pharmaceutical art, e.g. using one or more pharmaceutically acceptable diluents, carriers or excipients. "Pharmaceutically acceptable" has the meaning described hereinbefore. The nature of the composition and carriers or excipient materials, dosages etc. may be selected in a routine manner according to choice and the desired route of administration etc. Where variation is possible, dosages may likewise be determined in a routine manner and may depend upon the nature of the molecule, purpose of treatment, age of patient, mode of administration etc.

As set forth in the Examples, it has been found that use of TPCS₂ₐ at a dose (or concentration) of 0.05 to 0.5 mg/kg (or 0.1 to 0.5 mg/kg) (mg of agent per kg of body weight) provides particularly advantageous results. Conveniently a dose of 0.05 to 0.3 mg/kg (or 0.1 to 0.3 mg/kg), preferably 0.2 to 0.3 mg/kg is used. The photosensitizing agent may be rapidly or more slowly administered to the patient. Conveniently, TPCS₂ₐ may be administered over less than 30 seconds, e.g. from 1 to 15 seconds or over a longer time frame, e.g. 1 minute to 10 minutes.

"Gemcitabine" is a nucleoside analog having the structure indicated below (4-amino-1-(2-deoxy-2,2-difluoro-β-D-*erythro*-pentofuranosyl)pyrimidin-2(1*H*)-on) and encompasses pharmaceutically acceptable salts thereof.

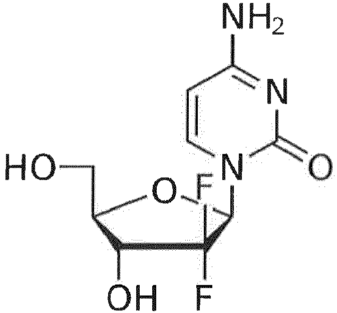

Gemcitabine works by inhibiting DNA synthesis and by inhibiting the enzyme ribonucleotide reductase, which is also involved in the cell's replication machinery. Gemcitabine is approved for several standard cancer chemotherapy regimens in indications such as ovarian cancer, breast cancer, non-small cell lung cancer and pancreatic cancer. Although not approved for this indication, it is also commonly used for palliative treatment of cholangiocarcinoma.

Gemcitabine is available from sources such as Eli Lilly & Co (Gemzar®) or Sigma-Aldrich, St. Louis, MO, USA. The pharmaceutically acceptable salt is preferably as defined hereinbefore, preferably the hydrochloride salt.

Gemcitabine is administered to the patient at a dose (or concentration) of 500-1500 mg/m² (which refers to mg of gemcitabine per m² of the body surface area, BSA). BSA may be calculated, for example, using the Mosteller formula (√([height(cm) x weight(kg)]/ 3600)). (Where necessary this may be converted to mg/kg by using a conversion factor for an average adult of 0.025mg/kg = 1 mg/m²). Conveniently a dose of 900-1100 mg/m² is used. Conveniently, gemcitabine may be administered over less than 1 hour, e.g. 15 to 45 minutes, e.g. around 30 minutes or over a longer time frame, e.g. from 1 hour to 12 hours.

"Irradiating" the cholangiocarcinoma refers to irradiation of the tumour with light as defined herein via an optical fiber. Irradiation may also be referred to herein as illumination. This serves to activate the photosensitising agent. The cells of the tumour may be illuminated directly (when in direct contact with the optical fiber) or indirectly (when located away from the optical fiber, through the screen of other cells), i.e. receive a light dose.

Illumination of the tumour or cells of the patient occurs 3-5 days after administration of the photosensitizing agent. Preferably the illumination occurs 4 days after that administration.

To ensure that the photosensitizing agent's activity affects the release of gemcitabine, irradiation is conducted just before, during and/or after gemcitabine administration. Gemcitabine has to be taken into the cell to be active, and in most cells various nucleoside transporters are important for its uptake into the cell. In some cancer cells the expression of such transporters may, however, be quite low, severely limiting the uptake of gemcitabine, and thereby the therapeutic effect that can be achieved. In such cells gemcitabine may, however, still be taken up by endocytosis, and the PCI-induced enhancement of the gemcitabine cytotoxic effect which has been observed (e.g. in the *in vitro* cancer cell studies described in the Examples) indicate that this may be an important transport mechanism in such cells.

Although not wishing to be bound by theory, it is likely that gemcitabine is taken up into intracellular compartments in the patient's cells and activation of the photosensitizing agent releases the gemcitabine into the cell. As a consequence, before irradiation is conducted the photosensitizing agent must be in the relevant compartments in the cell. It has been found that molecules in those compartments at the time of activation of the photosensitizing agent, or which are taken up into such cells immediately after such activation, are released into the cytosol. (See in this regard WO02/44396, which refers to the so-called "light before" method.)

Conveniently said irradiation is conducted within the time range of 1 hour before the start of gemcitabine administration up to 4, 5 or 6 hours after the start of gemcitabine administration. Preferably the irradiation is performed within 3 or 4 hours of the start of gemcitabine administration (i.e. from the start of gemcitabine administration to 3 or 4 hours after the start of gemcitabine administration, e.g. at time 0, 30, 60, 120, 180 or 240 minutes when time 0 is the start of gemcitabine administration). Thus, for example, when gemcitabine administration is conducted for 30 minutes, and irradiation is conducted for 15 minutes, the gemcitabine administration may be started at time 0, stopped at time 30 mins, irradiation started at time 120 minutes and concluded at time 135 minutes. However, irradiation performed up to 1, 2, 3 or 4 days after gemcitabine administration is also encompassed by the method of the invention.

Where appropriate the light irradiation step may be performed more than once (e.g. 2, 3 or 4 times). This may be necessary for particularly large tumours or discrete tumours spread over a wide area. In this case one or more optical fibers may be used which may be at the same or different locations. The location of the one or more optical fibers may be changed or maintained for each irradiation step (e.g. moved to a location close to other discrete tumours or areas of a single tumour mass).

The light irradiation step to activate the photosensitising agent may take place according to techniques and procedures well known in the art. The wavelength of light to be used is 640-665 nm, preferably 652 nm. Suitable artificial light sources are well known in the art. Conveniently, illumination (irradiation) is provided by the PCI Biotech AS 652nm laser system diode laser, although any suitable red light source may be used.

An optical fiber is used to provide the light which is placed within 3 cm of the CCA. As referred to herein an "optical fiber" is a thin, flexible, transparent fiber through which light can be transmitted at the distal end. Preferably the light is diffused to spread light evenly across a surface, minimizing or removing high intensity bright spots. Various tips at the end of the optical fiber may be used, e.g. a frontal distributor or microlense tip or a spherical diffuser. In the alternative a balloon catheter may be used to scatter the light (e.g. Medlight's diffusing balloon catheter). In another alternative the fiber may be unadorned, e.g. a bare tip may be used. Preferably the light is transmitted through a cylindrical diffuser in which the distal extremity of the fiber is an illumination tip which uniformly distributes the transmitted light along its length.

The fiber may be made of glass or plastic and has a core diameter which is at least the diameter of the laser output channel, preferably of 200 to 800 µm (e.g. 400-600, e.g. 500 µm) or a diameter of 400 to 1200 µm (e.g. 900 to 1000 µm) including any coating. Fibers with diffuser lengths of 10 to 70 mm, preferably 20 to 40 mm may be used. More than one optical fiber may be used for large tumours or multiple discrete tumours within a patient, e.g. 2 or more, e.g. 3, 4, 5, 6 or more, e.g. less than 10. In the alternative a single optical fiber may be used which may be moved to different locations, or maintained at the same location, for multiple rounds of illumination (irradiation).

In accordance with the invention the fiber (or at least one or each fiber) is placed within 3 cm of the CCA. This measurement refers to the distance between the exterior surface of the fiber and the closest portion of the CCA. Preferably the fiber is placed as close as possible to the CCA, e.g. within 1 or 2 cm of the CCA or within the CCA. Conveniently the fiber is placed in the bile duct (e.g. via a catheter).

The optical fiber may be provided by way of a catheter, e.g. in the form of an optical fiber based catheter which may be coupled with a laser source (i.e. such that the optical fiber is within the catheter during treatment and light irradiation from the fiber occurs through the catheter wall). The optical fiber guides the light from the proximal to the distal end of the device. The distal end preferably has an illumination tip ("diffuser") which may uniformly distribute light transmitted by the optical fiber along its length. The catheter allows endoscopic delivery of light. Preferably a light diffuser is used. Conveniently, Medlight SA (Switzerland) Cylindrical Light Diffusers may be used, e.g. the Cylindrical light diffuser Model RD with radiopaque markers.

The time for which the cells of the patient are exposed to light in the methods of the present invention may vary to achieve the required light dose. The time may be selected according to various factors including the dose of the photosensitizing agent and gemcitabine and fluence rate of the light to be used and the proximity of the optical fiber to the tumour.

The total light dose to the tumour cells may be expressed in J/cm of the fiber (or tumour) and is calculated as the fluence rate (W/cm of the fiber or tumour) x treatment time.

Depending on the fluence rate of the light source to be used, to achieve the desired light dose, the time of irradiation may be selected accordingly. For example, to achieve a light dose of 10 or 30 J/cm (or 15 or 30 J/cm) with a fluence rate of 100 mW/cm, an irradiation time of 2.5 minutes or 5 minutes, respectively, may be used. Thus, with a fluence rate of 100 mW/cm to achieve a light dose of 10 to 60 J/cm (or 15 to 60 J/cm) an irradiation time of 2.5 to 10 minutes may be used. A higher or lower fluence rate allows a lower or higher irradiation time to be used, respectively. Preferably, taking the above into account, the irradiation time is from 1 minute to 20 minutes, e.g. 2 to 10 minutes, depending on the fluence rate of the light source. These timings refer to each irradiation time when multiple rounds of illumination (irradiation) are used.

Appropriate light doses can be selected by a person skilled in the art and again will depend on the factors indicated above. In particular higher doses or concentrations of the photosensitizing agent (TPCS₂ₐ) allow a lower light dose to be used. In the present invention it has been found that light at a dose of 10 to 60J per cm (or 15 to 60J per cm) of the (diffuser of the) optical fiber or tumour is particularly advantageous. In the unit "J per cm" the cm is the length of the optical fiber if light is emitted over a length of the fiber (e.g. when a diffuser is used). In the alternative this may be cm of tumour, e.g. when a point light source is used. In both cases J/cm refers to the light dose provided per cm to the local environment. Conveniently the light dose is achieved using a light diffuser with a fluence of 100mW per cm of fiber (e.g. diffuser) and hence an irradiation time of from 2.5 to 10 minutes. Preferably a dose of 10 to 45 (or 15 to 45), e.g. 20 to 40 or 25 to 35 J/cm is used.

Furthermore, if cell viability is to be maintained, the generation of excessive levels of toxic species is to be avoided and the relevant parameters may be adjusted accordingly.

The methods of the invention may inevitably give rise to some cell damage by virtue of the photochemical treatment i.e. by photodynamic therapy effects through the generation of toxic species on activation of the photosensitizing agent. As the role of the method of the invention is to destroy tumour cells, this cell death may not be of consequence and may indeed be advantageous. In some embodiments, however, cell death should be avoided to ensure that the cytotoxic molecules are taken up into the cells to ensure localized and specific cell death. The methods of the invention may be modified such that the fraction or proportion of the surviving cells is regulated by selecting the light dose in relation to the dose (concentration) of the photosensitizing agent. Again, such techniques are known in the art.

Preferably, substantially all of the cells, or a significant majority (e.g. at least 75%, more preferably at least 80, 85, 90 or 95% of the cells) are not killed by the photochemical internalization method alone (i.e. without a cytotoxic agent). *In vitro* cell viability following PCI treatment can be measured by standard techniques known in the art such as the MTS test. *In vivo* cell death of one or more cell types may be assessed within a 1cm radius of the point of administration (or at a certain depth of tissue), e.g. by microscopy or other appropriate means. As cell death may not occur instantly, the % cell death refers to the percent of cells which remain viable within a few hours of irradiation (e.g. up to 4 hours after irradiation) but preferably refers to the % viable cells 4 or more hours after irradiation.

In optional methods of the invention, 1-40 days after step (ii), i.e. after administering the gemcitabine and subjecting the patient to irradiation, the patient may be systemically administered gemcitabine and/or another cytotoxic agent. Preferably such administration occurs 5-30 days, especially preferably 7-21 days after step (ii).

When gemcitabine is administered this may be administered as described hereinbefore for the gemcitabine administered in step ii) (i.e. as regards the dose, route and duration of administration).

The other cytotoxic agent may be any toxic agent which is suitable for treating a cancer, particularly cholangiocarcinoma with acceptable side-effects in the patient (e.g. commonly used chemotherapy drugs). Such agents include alkylating drugs, anthracyclines and other cytotoxic antibiotics (e.g. bleomycin), a protein toxin (e.g. gelonin), antimetabolites (not including gemcitabine), vinca alkaloids and etoposide, tyrosine kinase inhibitors and other antineoplastic drugs such as platinum compounds, e.g. cisplatin.

"Cisplatin" is a platinum containing anti-cancer drug with the structure indicated below ((*SP*-4-2)-diamminedichloroplatinum(II)).

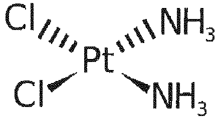

Cisplatin is available from sources such as Hospira (Cisplatin Hospira).

Appropriate doses for the other cytotoxic agents are known in the art. If cisplatin is used, conveniently it is used at a dose of 10-50 mg/m², preferably 20-30 mg/m². The route and duration of administration may be as described hereinbefore for gemcitabine.

In step iii) when gemcitabine and at least one other cytotoxic agent is used, they may be used simultaneously, separately or sequentially. When used simultaneously they are administered at the same time, but may be administered by a single route or via separate routes (e.g. a mixture administered intravenously or two (or more) preparations administration at the same time but via different intravenous entry points). When administered separately they may be administered at the same time or sequentially and/or may overlap in their administration timing. When used sequentially the time separating administration of the different agents is not more than 24 hours. Conveniently they are administered together in a single mixture.

In preferred features of the invention the gemcitabine and/or another cytotoxic agent used in step iii) is administered more than once, e.g. 2, 3, 4, 5, 6, 7, 8, 9 or 10 times (e.g. up to 20 times). This administration may be in a single (or each) cycle or in total in multiple cycles.

As referred to herein a "cycle" is a time period over which a particular treatment regime is applied and is generally repeated to provide cyclical treatment. The treatment in each cycle may be the same or different (e.g. different dosages, timings etc. may be used). A cycle may be from 14-30 days in length, e.g. a 21 day cycle. Multiple cycles may be used, e.g. at least 2, 3, 4 or 5 cycles, e.g. 6, 7, 8, 9 or 10 (e.g. up to 8, 9, 10 or 20) cycles. Within each cycle the gemcitabine and/or another cytotoxic agent may be administered once or more than once, as described hereinbefore. In a preferred feature the gemcitabine and/or another cytotoxic agent is administered twice in each cycle and preferably at last 3, preferably at least 5 cycles are conducted.

Whilst in a preferred feature the above step iii) treatment with gemcitabine and/or another cytotoxic agent is performed, in another aspect of the invention such treatment is not performed and in particular the treatment does not include the use of another cytotoxic agent such as cisplatin.

For effective treatment, the method described herein, or a part thereof, may be repeated. Thus, for example, steps i) and ii) may be repeated one or more times, e.g. 2 or 3 times, for example after an interval of from 1 to 6 months or longer, e.g. after step ii) or iii). In a preferred aspect the entire method is repeated at least twice, e.g. 3 or 4 times.

In a particularly preferred aspect the method comprises performance of the method twice (i.e. performance of steps i), ii), iii), i), ii) and iii)) or more, preferably in which in the first and/or second (or later) round of steps i) to iii), step iii) is performed at least twice. Thus by way of example, the method may be performed as follows:
i) systemically administering TPCS₂ₐ to said patient to a dose of 0.05 to 0.5 mg/kg (or 0.1 to 0.5 mg/kg) and
ii) after 3-5 days, systemically administering gemcitabine to a dose of 500-1500 mg/m² and 2-4 hours after completion of the gemcitabine administration irradiating said cholangiocarcinoma with light with a wavelength of 640-665nm using an optical fiber placed within 3cm of said cholangiocarcinoma to provide a light dose of 10 to 60 J/cm (or 15 to 60 J/cm);
iii) after 1-40 days (preferably after 7-21 days) systemically administering gemcitabine and/or another cytotoxic agent, preferably cisplatin;
iv) repeating step iii) at least once; and
v) repeating steps i) to iv) at least once.

In a particularly preferred aspect, step iii) is performed in cycles as described hereinbefore, in particular multiple cycles of step iii) are performed (before commencing to step iv)) and in each of these cycles the gemcitabine and/or another cytotoxic agent such as cisplatin is administered twice or more.

By way of example the method may be performed in which step iii) in the first and second round comprises 2, 3 or 4 cycles of gemcitabine and cisplatin administered once or twice, e.g. at days 1 and 8. When steps i) and ii) are performed after the first round step iii) and before the second round step iii), it may impinge on the first cycle in step iii) of the second round, e.g. the gemcitabine administration and irradiation may replace the first administration of gemcitabine and cisplatin in the first cycle. In one option, the patient is not subjected to any treatment for a period of between 1 week to 4 weeks after the first round (of steps i), ii) and iii)) before the second round is commenced. In that case the treatment then resumes with steps i) and ii) as indicated above, followed by step iii). Preferred timings of the treatment and/or doses to be used are as described in the Examples.

Prior to treatment the patient to be treated may be subjected to biliary stenting to ensure adequate biliary drainage. The stent, which may be plastic or metal, is placed into the bile duct using procedures known in the art. The latter may be kept in place during illumination whereas the former are removed during illumination and a new stent inserted after that procedure.

The present invention also provides a kit comprising gemcitabine and TPCS₂ₐ and optionally another cytotoxic agent as defined hereinbefore, for simultaneous, separate or sequential use to treat a cholangiocarcinoma in a patient, wherein said use is as defined hereinbefore.

Preferred aspects according to the invention are as set out in the examples.

The invention will now be described in more detail in the following non-limiting examples with reference to the following drawings in which:

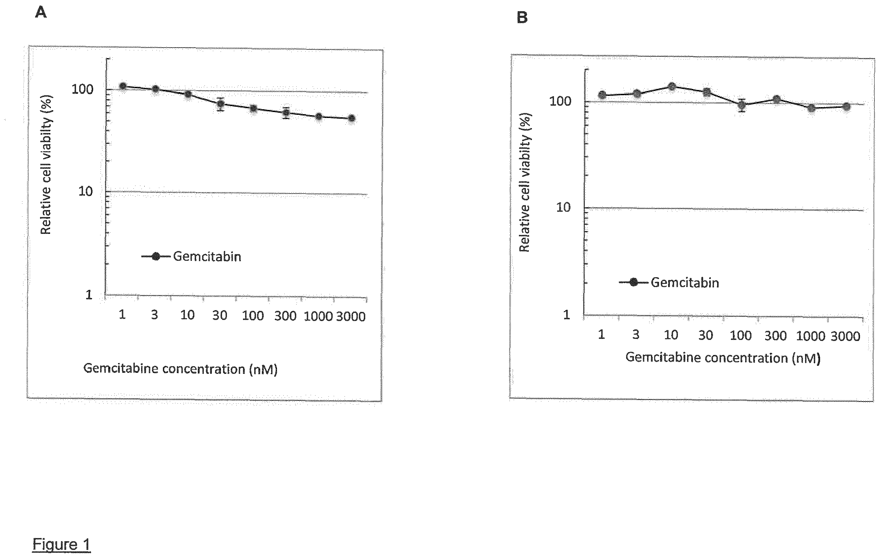

**Figure 1** shows cytotoxicity after 72 hours gemcitabine exposure. Viability of the cell lines TFK-1 (A) and EGI-1 (B) were analysed by the MTT assay after 72 hours gemcitabine incubation as described under Materials and Methods in Example 1. Each data point represents the mean (+/- standard deviation) from three experiments.

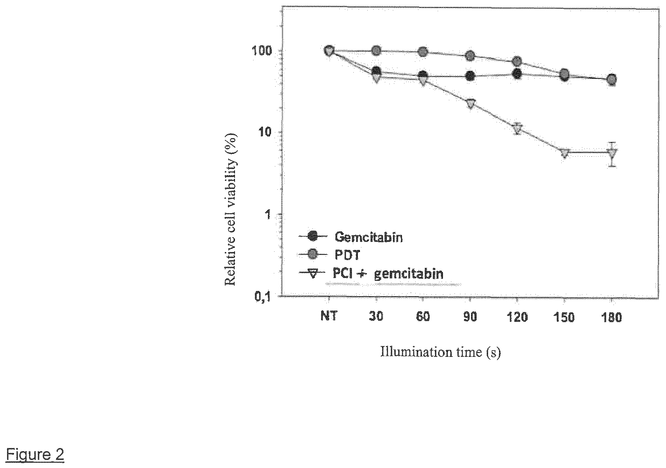

**Figure 2** shows PCI with 100 nM gemcitabine in the TFK-1 cell line. The experiment was performed with a gemcitabine dose of 100 nM as described under Materials and Methods in Example 1. The data points are mean values of 3 parallel measurements (+/- standard deviation) and represent one representative of 3 independent experiments. PDT in the figure means "PCI alone".

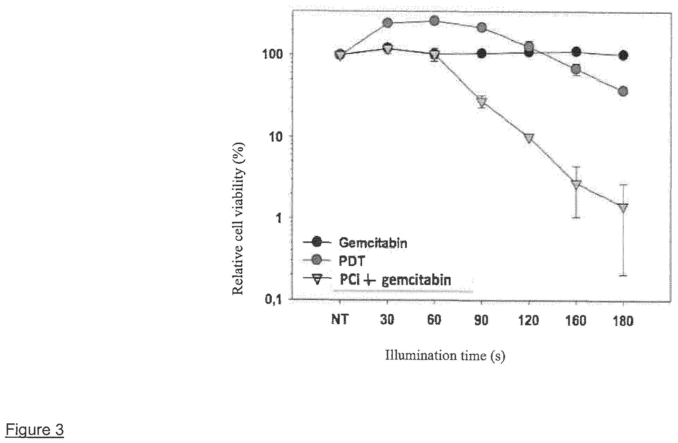

**Figure 3** shows PCI with 100 nM gemcitabine in the EGI-1 cell line. The experiment was performed with a gemcitabine dose of 100 nM as described under Materials and Methods in Example 1. The data points are mean values of 3 parallel measurements (+/- standard deviation) and represent one representative of 3 independent experiments. PDT in the figure means "PCI alone".

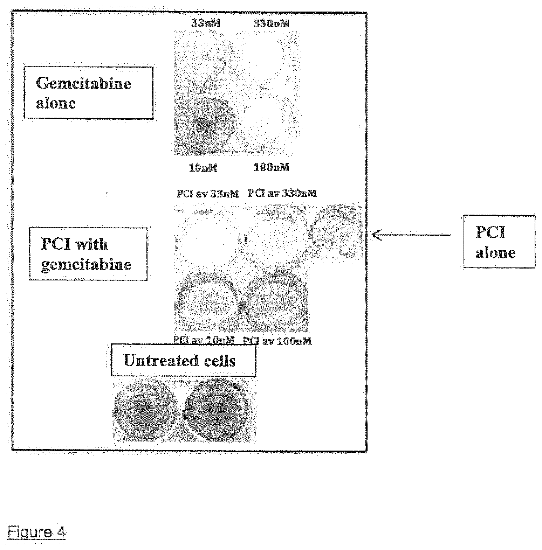

**Figure 4** shows colony forming ability of TFK-1 cells after PCI treatment with different doses of gemcitabine. The experiment was performed as described under Materials and Methods in Example 1 with different doses of gemcitabine (indicated on the figure) and an illumination time of 140 s. Three independent experiments were performed with essentially similar results.

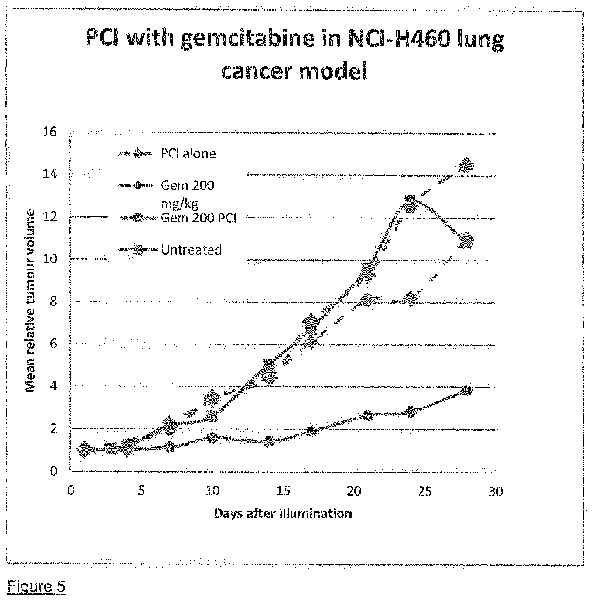

**Figure 5** shows the results of an animal study on the effect of PCI with 200 mg/kg gemcitabine. Animals with subcutaneously growing NCI-H460 human lung cancer tumour cells were administered 5 mg/kg Amphinex® (TPCS₂ₐ) by intravenous injection. 3 days later the animals were administered 200 mg/kg of gemcitabine, and the tumours were illuminated 4 h later. The size of the tumours was measured 2-3 times per week. The results show that neither the photochemical treatment alone (PCI alone) nor gemcitabine alone had a significant effect on tumour growth as compared to a control group with untreated tumours (Untreated). In contrast, the combination of PCI and gemcitabine substantially reduced tumour growth indicating the PCI can significantly enhance the effect of gemcitabine.

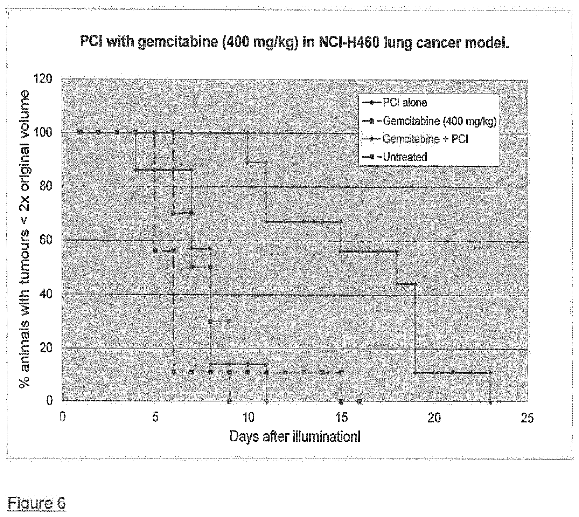

**Figure 6** shows the results of an animal study on the effect of PCI with 400 mg/kg gemcitabine. The method was performed as described above for Figure 5 except that 400 mg/kg of gemcitabine was used. The results show that neither the photochemical treatment alone (PCI alone) nor gemcitabine alone (Gemcitabine 400 mg/kg) had a significant effect on tumour growth as compared to a control group with untreated tumours (Untreated). In contrast, the combination of PCI and gemcitabine substantially reduced tumour growth, indicating the PCI can significantly enhance the effect of gemcitabine.

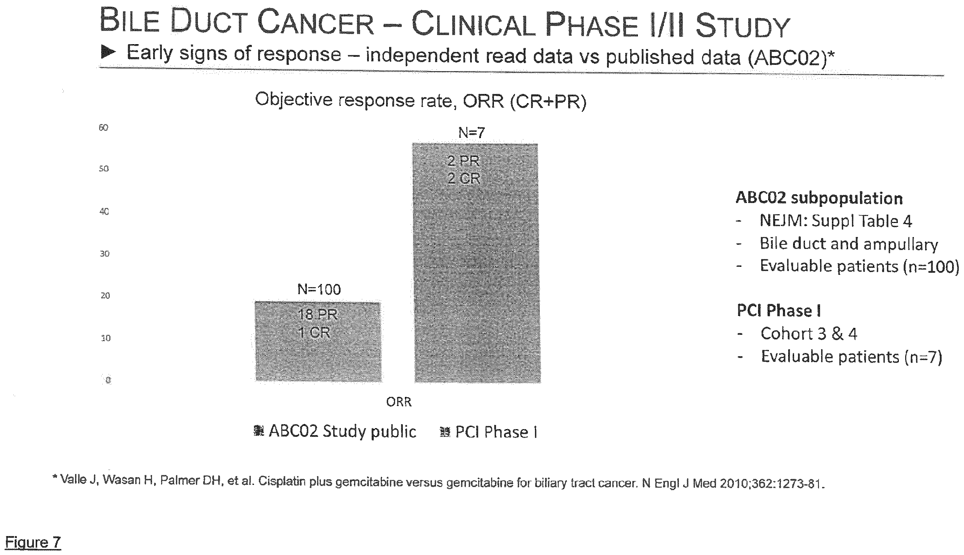

**Figure 7** shows the percent of patients which exhibited a positive response to treatment at 6 months, i.e. those with a partial response (PR) or complete response (CR). Patients subjected to PCI treatment (PCI Phase 1 : cohorts 3 and 4) compared to those treated with a standard non-PCI chemotherapy treatment (ABC02).

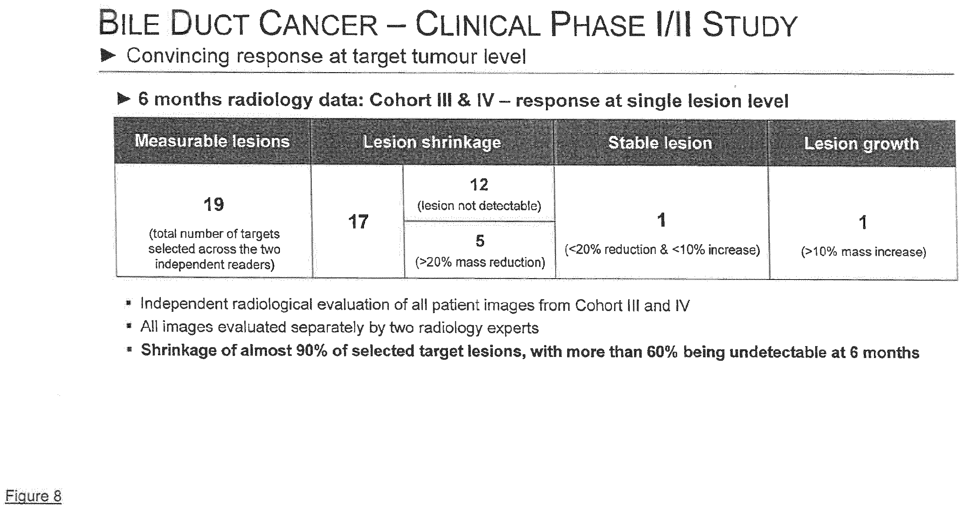

**Figure 8** shows the response of cohort 3 and 4 cholangiocarcinoma patients in terms of the status of the sum of all target (measurable) lesions after PCI treatment.

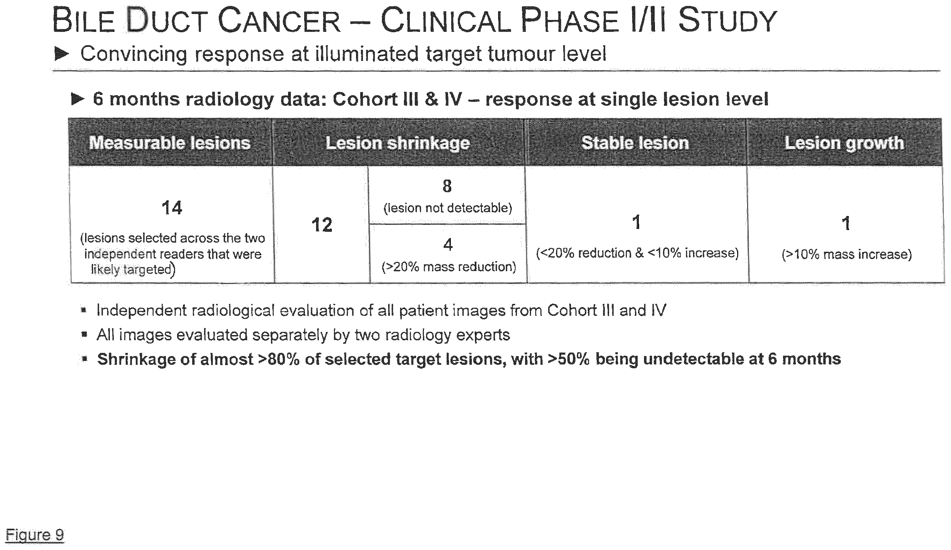

**Figure 9** shows the response of cohort 3 and 4 cholangiocarcinoma patients in terms of the status of the anticipated treated target (measurable) lesions after PCI treatment.

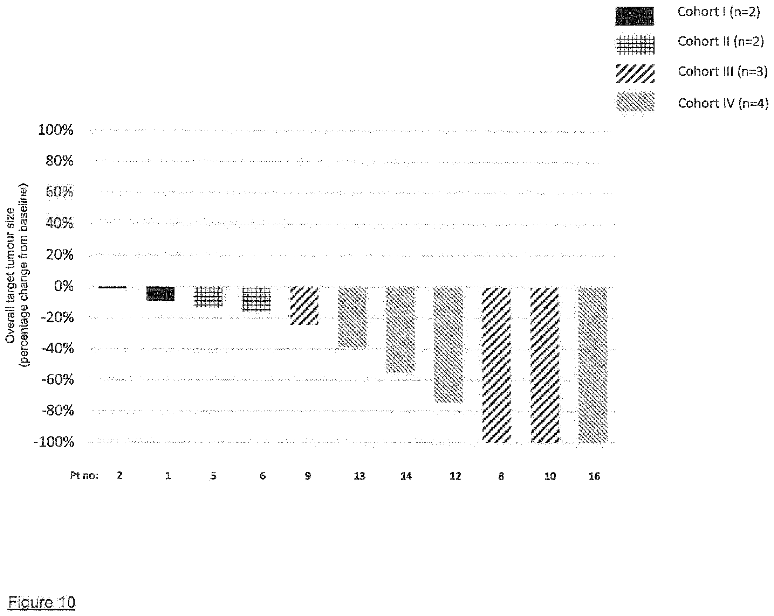

**Figure 10** shows the overall target tumour size reduction in all radiologically evaluable cholangiocarcinoma patients from all the cohorts.

### EXAMPLE 1: In vitro evaluation of PCI with gemcitabine in cholangiocarcinoma cells

### MATERIALS AND METHODS

### Cell lines and growth media

The cell lines used were TFK-1 and EGI-1, both were obtained from the Department of Cancer Prevention, The Norwegian Radium Hospital, Oslo, Norway. TFK-1 is a human papillary bile duct adenocarcinoma cell line and EGI-1 is a poorly differentiated human bile duct adenocarcinoma cell line. Both cell lines originate from extrahepatic tumours (Saijyo et al., 1995, Tohoku J. Exp. Med., 177:61-71; EGI-1 - Cell LINCS Library of Intergrated network-based Cellular Signatures. Available from: http://lincs.hms.harvard.edu/db/cells/50181/.), but are quite different both morphologically and physiologically (Xu et al., 2013, Clinical Cancer Research, Jan 1;19(1):118-27; Pignochino et al., 2010, BMC Cancer, 10:631).

The cells were grown as monolayer cultures in 75 cm² tissue culture flasks (NUNC, Thermo Fisher Scientific, Roskilde, Denmark) at 37 °C with 5% (v/v) CO₂. The TFK-1 cell line was grown in RPMI-1640 medium (Sigma-Aldrich, St. Louis, MO, USA) with L-glutamine, 10% fetal calf serum (FCS) (PAA Laboratories, Pasching, Austria), 100 U/ml penicillin and 100 µg/ml streptomycin (Sigma-Aldrich). The EGI-1 cell line was grown in DMEM medium (Sigma-Aldrich) with L-glutamine, 10% FCS, 100 U/ml penicillin and 100 µg/ml streptomycin.

### TPCS₂ₐ photosensitiser

Di (monoethanolammonium) meso-tetraphenyl chlorin disulfonate (TPCS₂ₐ/Amphinex®) was provided by PCI Biotech AS (Lysaker, Norway). Amphinex batch number: FAMP 1002. TPCS₂ₐ (in 3% polysorbate 80, 2.8% mannitol, 50 mM Tris pH 8.5) stock solution (0.4 mg/ml) was kept at 4°C in aliquots and light protected. All work with the photosensitizer was performed under subdued light.

### Light source

Illumination of the cells was performed by using Lumisource (PCI Biotech, Oslo). This lamp consists of four standard light tubes (18 W/tube, Osram L 18/67), which emit blue light with a main peak at approximately 435 nm and was used for excitation of TPCS₂ₐ. The irradiance emitted from the blue light source was 12 mW/cm² and varied less than 10% across the illumination area (765 cm²). The light source was air-cooled during light exposure to keep the irradiance stable and prevent cells from hyperthermia.

### Cytotoxicity of gemcitabine without PCI

The cells were incubated with different concentrations of gemcitabine (1-1000 nM) for 72 hours and cell viability was assessed by the MTT assay (see below) as described earlier for these cell lines (Pignochino *et al.,* 2010, *supra*; Lieke et al., 2012, BioMed Central, 12:1471-2407).

### PCI with gemcitabine

Cells were seeded in 96-well or 6-well plates (Nunclon, Nunc, Roskilde, Denmark) and allowed to attach at 37 °C overnight. Gemcitabine was added to the cells and incubated for 54 hours before the addition of 0.4 µg/ml TPCS₂ₐ. After 18 h of further incubation the cells were washed in TPCS₂ₐ-free medium, resuspended in TPCS₂ₐ-free medium with gemcitabine and incubated for 4 h. The cells were illuminated, washed and resuspended in drug-free medium, and cell viability was assess by the MTT assay 48 hour after illumination, or by the colony forming assay 6 days after illumination (see below).

### MTT cell viability assay

The MTT method (tetrazolium dye reduction) was performed 48 h after light exposure with Lumisource. The culture medium was removed and cells were incubated in medium containing 0.25 mg/ml MTT (Sigma) for approximately 3 hours before replacement with 100 µl of 99% dimethylsulfoxide (Sigma). The 96 well plates were set on a shaker for 5 minutes before absorbance was measured at 570 nm with a power wave XS2 (Biotek, VT, USA). Wells without cells incubated with MTT-medium only were used for blank subtraction.

### Colony formation assay

Cells (25 000 cells/well) were seeded out in 6 well plates (Nunc) and allowed to adhere overnight and subjected to photochemical treatment described above. The cells were placed in the incubator and the culture medium was replaced with fresh culture medium after 3 days incubation. The colonies were washed once with 0.9% mg/ml NaCl, fixed in 96% ethanol for 10 min, and stained with a saturated solution of methylene blue (Sigma) for 10 min. Subsequently, cells were washed in H₂O and then dried before being evaluated by visual inspection.

### RESULTS AND DISCUSSION

### Cytotoxicity of gemcitabine in the TFK-1 and EGI-1 cell lines

In initial experiments cytotoxicity was investigated with a gemcitabine incubation time of 4 hours, since with several other drugs this is a suitable incubation time in PCI experiments. The results showed, however, that with 4 hours exposure even at very high doses (up to 1 mM was tested) gemcitabine had only a very modest cytotoxic effect on these cell lines (data not shown). It was therefore decided to increase the gemcitabine exposure time to 72 hours, which has been used earlier for these cell lines (Pignochino *et al.,* 2010, *supra*; Lieke *et al.,* 2012, *supra*).

The cytotoxicity of gemcitabine in concentrations up to 3 µM with 72 hours exposure is shown in Figure 1. It can be seen that even with 72 hours incubation gemcitabine cytotoxicity was rather modest, reaching about 50 % cytotoxicity at 3 µM gemcitabine for the TFK-1 cell line, while the EGI-1 cell line was insensitive to gemcitabine even at the highest dose employed in this experiment. It was decided to use a gemcitabine concentration of 100 nM in the PCI experiments, since at this dose the cytotoxicity of gemcitabine alone was at most modest and would not obscure the effects of the PCI treatment.

### PCI with gemcitabine with the MTT cytotoxicity assay

The experiment was performed with a gemcitabine dose of 100 nM as described under Materials and Methods. From Figure 2 it can be seen that in the TFK-1 cell line PCI significantly increased the cytotoxic effect of gemcitabine. Thus, while gemcitabine alone gave a cytotoxic effect of about 50 % (in good accordance with the results shown in Figure 1), the combination with PCI strongly increased cytotoxicity in a light dose dependent manner. The photochemical treatment alone (PDT in Figure 2) had only a modest effect on cell survival, indicating that PCI synergistically increases the cytotoxicity of gemcitabine.

The same conclusion could be drawn from the experiments with the EGI-1 cell line (Figure 3). In this cell line gemcitabine alone had no effect on cell survival (again in accordance with the results in Figure 1). However, with PCI a very strong cytotoxic effect was achieved (99% cell killing) while the photochemical treatment alone (PCI alone) at the highest light dose killed only about 50% of the cells. In this cell line with PCI a very good cytotoxic effect was also achieved under conditions where neither gemcitabine nor PCI alone (PDT in the figure) had any cytotoxic effect (e.g. 120 s illumination in Figure 3), clearly showing the synergistic effect of PCI with gemcitabine. It is also worth noting that with PCI > 90 % cell killing could be achieved with a dose of gemcitabine of 100 nM, while without PCI this level of cell killing was not obtained even with a dose 30 times higher (3 000 nM dose in Figure 1B).

### PCI effect on colony forming ability of the TFK-1 cell line

TFK-1 cells were treated with PCI and cell viability was assessed by the colony forming assay as described under Materials and Methods. Initial experiments showed that gemcitabine alone has a substantially stronger effect on the colony forming ability than on viability as assessed by the MTT assay (see also Figure 4). In this experiment it was therefore necessary to use also lower doses of gemcitabine. From Figure 4 it can be seen that after treatments with gemcitabine doses of 330 and 100 nM the TFK-1 were unable to form colonies, at 33 nM some colony formation could be observed, while at 10 nM substantial colony formation was observed, albeit still less than what was observed in untreated samples. With 10 nM gemcitabine + PCI colony formation was not observed, while in samples treated with PCI alone the cells still had the ability to form colonies. This strongly indicates that PCI enhances the effect of gemcitabine also on the cells' ability to form colonies, something that is of course very important for use in cancer treatment, since this is a measure of the cells' ability to continue growing after the treatment.

### CONCLUSION

The results show that photochemical internalization can significantly enhance the effect of gemcitabine on two different cholangiocarcinoma cell lines that are quite resistant to the effect of gemcitabine when used without PCI.

### EXAMPLE 2: In vivo (mice) evaluation of PCI with gemcitabine to provide an anti-cancer effect

This study was used to assess the *in vivo* anti-cancer efficacy of photochemical internalisation (PCI) with the photosensitiser TPCS₂ₐ used in combination with the cytotoxic agent gemcitabine.

### MATERIALS

| | |
|---|---|
| Test substance 1: | Amphinex (TPCS₂ₐ) (batch no. FAMP 1002, PCI Biotech AS) |
| Test substance 2: | Gemcitabine (Sigma-Aldrich) |
| Vehicle for Amphinex: | 3 % Tween-80; 2.8 % mannitol; 50 mM Tris pH 8.5 |
| Vehicle for gemcitabine: | 0.9 % NaCl |

### MICE, ROUTE OF ADMINISTRATION AND DOSE LEVELS

Nude (nu/nu) athymic mice (female, CD-1 nu/nu strain, at least 6 weeks old) were used which have been used extensively to generate human tumour xenografts and remain the experimental method of choice for testing anti-tumour efficacy of new compounds prior to administration in man. The route of administration of the test substances was intravenous. The dose of Amphinex® was 5 mg/kg, based on earlier mouse studies performed by PCI Biotech AS and collaborators.

### METHODS

Amphinex® was formulated for dosing by diluting in 3 % Tween-80; 2.8 % mannitol; 50 mM Tris pH 8.5 to provide a 1.25 mg/mL dosing solution.

Gemcitabine was formulated for dosing by dissolving in 0.9 % NaCl to give dosing solutions of 20 and 40 mg/mL.

Solutions were freshly prepared on the day of dosing and protected from light.

There were 6 treatment groups with 10 mice per group. Mice were injected subcutaneously with 7 x 10⁷ cells/mL NCI-H460 tumour cells to allow selection of optimal tumours for inclusion in the study. Tumours of the appropriate size were allocated to the various treatment groups.

Each group was given a number (1 to 6). The treatment groups were the following:

| | |
|---|---|
| Group 1 | Gemcitabine; 200 mg/kg |
| Group 2 | Amphinex® + Gemcitabine + laser treatment; 5 mg/kg + 200 mg/kg |
| Group 3 | Vehicle Group + Amphinex® + laser treatment; 5 mg/kg |
| Group 4 | Vehicle group + no laser treatment; |
| Group 5 | Gemcitabine; n = 10; 400 mg/kg |
| Group 6 | Amphinex® + Gemcitabine + laser treatment; 5 mg/kg + 400 mg/kg |

Amphinex® was administered intravenously via a tail vein, using a dose volume of 4 mL/kg.

The route of administration of gemcitabine was intravenous at a dose volume of 10 mL/kg.

The vehicle group was 0.9% NaCl and this was dosed by intravenous injection at a dose volume of 10 mL/kg.

Approximately 4 h post gemcitabine or vehicle administration, animals in Groups 2, 3 and 6 had their tumours illuminated with a 650 nm diode laser (see below).

### Procedure

Human NCI-H460 non-small cell lung cancer cells (American Type Culture Collection [ATCC], Maryland, USA, or the European Collection of Cell Cultures [ECACC], Porton Down, UK) were harvested from sub-confluent cultures growing *in vitro* and the number of viable cells determined. Cells were then re-suspended in sterile phosphate buffered saline (PBS) at a concentration of approximately 7 x 10⁷ cells/mL. Nude (nu/nu) athymic mice were injected subcutaneously in the right flank with approximately 7 x 10⁶ NCl-H460 cells in a volume of 0.1 mL. Animals were examined regularly for the appearance of tumours. When measurable tumours had established in the majority of mice, animals were assigned into 6 treatment groups with a target of up to 10 mice per group. Mice received an intravenous injection of Amphinex when the tumours had reached a size such that the tumour volume was approximately in the range 100 to 150 mm³ 72 hours later. At this time point the animals were dosed with gemcitabine, and the tumours were illuminated approximately 4 hours later.

For tumour illumination each animal was restrained, one at a time, in a metal restraint which allows only the lower flank area, i.e. tumour area plus a 2 mm diameter surrounding area, to be exposed. A 650 nm diode laser (TWI Diode Laser, Quanta System, Italy) was then used to expose the tumour tissue to a light dose of 15 J/cm², with a fluence rate of 90 mW/cm². Each animal had their tumour exposed to the laser for approximately 167 s, to give the required dose of 15 J/cm².

If the tumour sizes varied by large amounts, then the fluence rate at the surface of the animal was adjusted to 90 mW/cm², to give the correct dose and illumination time for each animal. For example, for a light dose of 15 J/cm², a fibre output of 120 mW is required to cover an illumination diameter of 1.3 cm (an approximate average tumour diameter), illumination time is 167 s and the distance of the fibre to the tumour should be 2.1 cm.

Tumour size was measured at least twice weekly using digital callipers for up to 4 weeks following illumination or until the tumour size (as specified in the UK Home Office Licence) or other clinical signs necessitated removal of that mouse from the study. Tumour dimensions were recorded (length and width), and tumour volumes calculated using the formula (W² x L) / 2, where W is the widest tumour dimension and L is the longest. Animals were terminated if the tumour size had become excessive or any adverse effects were noted.

### DATA ANALYSIS

The tumour dimensions measured over the period of the study - length (L, long) and width (W, short) in mm, were recorded and kept as raw data. The mean tumour volumes on the first day of treatment were reported for each group. Calculations of relative tumour volumes and plots of mean tumour growth curves were performed. Body weight data were also obtained.

### RESULTS AND DISCUSSION

The results of the study showed that the PCI technology can significantly enhance the effect of gemcitabine *in vivo* (Figure 5). It can be seen that the pure Gemcitabine treatment had no (Figure 5, 200 mg/kg gemcitabine) or only a very modest (Figure 6, 400 mg/kg gemcitabine) effect on tumour growth as compared to an untreated control group. It can also be seen that the photochemical treatment alone (PCI alone, i.e. Amphinex® and laser light application without gemcitabine) also had virtually no effect. However, the combination of gemcitabine and PCI led to a substantial delay in tumour growth with both gemcitabine doses, inducing a clear synergistic effect.

The body weights of the animals in the study are shown in Table 1. All experimental groups included 10 animals at the start of the study, but during the study some animals had to be sacrificed, mainly due to excessive growth of the experimental tumour. The number of animals having to be sacrificed was larger in the control groups (i.e. "untreated" and "PCI alone" groups) and lowest in the groups receiving PCI with gemcitabine, since the latter treatment induced a significant delay in tumour growth. It can be seen that in all experimental groups the animals had, on average, gained weight at the end of the study. Apparently, the weight gain was largest in the "PCI alone" control group, which had the lowest average body weight at the start of the study. Between the other groups the weight gain was quite similar. In some of the groups a slight reduction in the body weight (maximum 7 %) was seen in the first period after the time point of illumination. This reduction was most pronounced in the groups receiving PCI with gemcitabine treatment, but a possible short-lived weight reduction was also observed in some of the other groups. These data suggest that there is no obvious or irreversible worsening of the general toxicity of gemcitabine by either Amphinex or the PCI treatment.

**Table 1: Body weight of animals treated with PCI with gemcitabine.**

| **Day** | **0** | **3** | **7** | **10** | **14** | **17** | **21** | **24** | **28** | **No. of animals alive at the end of study** |
|---|---|---|---|---|---|---|---|---|---|---|
| **Gemcitabine 200 mg/kg** | 100 | 101 | 104 | 105 | 106 | 109 | 111 | 111 | 114 | 5 |
| | 22.8 ± 1.2 | 23.1 ± 1.1 | 23.7 ± 1.3 | 23.9 ± 1.5 | 24.1 ± 1.5 | 24.8 ± 1.7 | 25.4 ± 1.1 | 25.3 ± 1.0 | 26.0 ± 1.6 | |
| **Gemcitabine 200 mg/kg PCI** | 100 | 97 | 93 | 94 | 96 | 101 | 103 | 101 | 105 | 6 |
| | 25.4 ± 2.4 | 24.6 ± 2.3 | 23.7 ± 2.1 | 23.9 ± 1.9 | 24.4 ± 2.1 | 25.7 ± 1.9 | 26.0 ± 2.2 | 25.7 ± 1.3 | 26.6 ± 1.2 | |
| **Untreated** | 100 | 99 | 99 | 99 | 99 | 102 | 103 | 103 | 110 | 3 |
| | 24.1 ± 2.3 | 23.9 ± 2.1 | 23.9 ± 1.9 | 23.9 ± 2.0 | 23.8 ± 2.0 | 24.6 ± 2.0 | 24.9 ± 1.6 | 24.9 ± 0.9 | 26.5 ± 0.2 | |
| **PCI alone** - | 100 | 98 | 104 | 103 | 112 | 109 | 113 | 117 | 126 | 2 |
| | 21.5 ± 1.6 | 21.1 ± 2.1 | 22.3 ± 1 .3 | 22.1 ± 1.2 | 24.1 ± 1.2 | 23.4 ± 1.3 | 24.4 ± 1.3 | 25.1 ± 1.3 | 27.0 ± 1.1 | |
| **Gemcitabine 400 mg/kg** | 100 | 96 | 102 | 104 | 106 | 108 | 109 | 109 | 107 | 6 |
| | 23.6 ± 2.0 | 22.6 ± 2.4 | 24.0 ± 2.1 | 24.4 ± 2.2 | 25.1 ± 2.3 | 25.4 ± 2.3 | 25.8 ± 2.5 | 25.8 ± 2.2 | 25.2 ± 1.8 | |
| **Gemcitabine 400 mg/kg PCI** | 100 24.5 ± 2.7 | 93 22.7 ± 2.6 | 95 23.4 ± 2.1 | 99 24.1 ± 2.2 | 101 24.7 ± 2.2 | 104 25.4 ± 2.3 | 104 25.5 ± 2.0 | 106 26.1 ± 2.1 | 109 26.7 ± 2.5 | 9 |
| | | | | | | | | | | |

Female nude (nu/nu) athymic CD-1 mice were subcutaneously transplanted with human NCI-H460 lung cancer cells. When the tumours had reached a volume of about 100 mm³ the mice were administered TPCS₂ₐ (5 mg/kg) by intravenous (i.v.) injection. 4 days later gemcitabine (doses specified in the table) was administered by i.v. injection, and 4 h later the tumours were illuminated with 650 nm light at a dose of 15 J/cm². The tumour size and the body weight of the animals were recorded at different time points after illumination (day 0 = day of illumination). The treatment groups are indicated in the table; the upper row for each treatment group is the average body weight in percentage of the weight at day 0; the lower row is the measured body weights (in grams) ± standard deviation.

### CONCLUSION

The results show that PCI with the Amphinex photosensitiser can significantly enhance the anti-tumour effect of gemcitabine in the NCI-H460 lung cancer model in nude mice.

### EXAMPLE 3: In vivo (humans) evaluation of PCI with gemcitabine in treatment of cholangiocarcinoma

The study was a phase I/II dose escalation study to assess the safety, tolerability and efficacy of Amphinex®-induced photochemical internalisation (PCI) of gemcitabine followed by gemcitabine/cisplatin chemotherapy in patients with advanced inoperable cholangiocarcinomas.

### Study population:

Male and female patients with histologically or cytologically diagnosed inoperable, locally advanced or metastatic, cholangiocarcinoma who were chemo-naïve.

### Drug:

Amphinex 30 mg/ml: 30 mg/ml of the active substance TPCS₂ₐ.2MEA (TPCS₂ₐ.2(monoethanolamine)) (26 mg/ml of the active moiety TPCS₂ₐ) Excipients: TPCS₂ₐ.2MEA is formulated in 3.0 % Tween 80, 50 mM Tris buffer pH 8.5 and 2.8 % Mannitol

### Treatment:

### Stenting:

All patients had biliary stenting prior to PCI treatment, to ensure adequate biliary drainage.

A key exclusion parameter reflecting this is the 'Serum (total) bilirubin', which could be up to max 1.5 x the Upper Limit of Normal (ULN) for the hospital.

Stents could be either plastic or metal. If plastic stenting: this/these was/were removed during the ERCP procedure prior to laser illumination (Day 4), and new stent(s) put in place right after the illumination procedure. If metal stents were used: these were kept in place in the bile duct also during laser illumination.

### PCI treatment

Day 0: A single dose of Amphinex® (fimaporfin) administered systemically by intravenous injection (dose escalation - see below) in a less than one ml volume which was flushed through with 0.9% NaCl.

Day 4: A single dose of Gemcitabine, 1000 mg/m², reconstituted and diluted with 0.9 % sodium chloride in accordance with the SmPC/Prescribing Information, administered by intravenous infusion over 30 minutes.

Day 4 - Laser light application: performed within a time window of 3 hours (±1 hour) after start of the gemcitabine administration. Laser light application was performed using the CE-marked PCI 652 nm red light laser, irradiance 100 mW/cm (i.e. 100 mW per cm diffuser length of the optical fibre) to achieve the indicated light dose (e.g. in cohort 1 patients were subjected to 150 sees illumination to achieve a 15J/cm dose and cohort 2-4 patients were subjected to 300 sees illumination to achieve a 30 J/cm dose, but some patients with larger or more tumours required additional illuminations). (dose escalation - see below)

Optical fibre: Cylindrical light diffuser Model RD, with active diffuser lengths of 2, 3 or 4 cm. Supplier: Medlight SA, Switzerland (http://www.medlight.com/). If there were more than one tumour, or the tumour was larger than the active diffuser tip of the fibre, additional illuminations could take place, in order to cover the entire/all tumour(s).

Catheter for positioning of the optical fibre (the optical fibre was pushed into this catheter, so the active distal diffuser tip of the fibre could be placed at the tumour site(s): Under fluoroscopic guidance the translucent ERCP catheter was advanced into the bile duct. ERCP-catheters used: MTW ERCP-catheter, with a metal tip, length 215 cm, Ø = 2.3 > 1.8 mm, article no. 99 01 30 31 2, or MTW ERCP-catheter 035 Conical metal tip cannula, length 215 cm, Ø = 1.8 mm. Article No. 01 30 61 1. Supplied by MTW Endoskopie, Germany (http://nmg.kiev.ua/fites/Katalog%20MTW.pdf).

In one patient in cohort 1 (patient 2), a second PCI treatment was conducted approximately 9 months after the first PCI treatment using the same dose parameters (Amphinex 0.06mg/kg and a light dose of 15 J/cm). Patient 6 in cohort 2 also received a second PCI treatment approximately 17 months after the first PCI treatment using Amphinex at 0.25 mg/kg and a light dose of 30 J/cm.

### Background treatment (standard treatment)

Commencing between 7 and 21 days after the laser light application, all except 4 patients received 8 cycles of standard systemic chemotherapy: cisplatin (25 mg/m²) and gemcitabine (1000 mg/m²), given on Day 1 and on Day 8 of each 21-day cycle.

In particular the patient cohorts received the following background treatments:
Cohort 1: 3 patients - 2 received 8 cycles; 1 received cycles 1 and 2 and the first chemotherapy administration (Day 1) of cycle 3
Cohort 2: 3 patients - all 3 received 8 cycles
Cohort 3: 4 patients - 3 patients received 8 cycles; 1 received the first chemotherapy administration (Day 1) of cycle 1
Cohort 4: 6 patients - 4 patients received 8 cycles; 1 received 6 cycles; 1 did not receive any cycles

### Dose Escalation - Phase I

The following doses of Amphinex and light were explored in a total of 16 patients. A cohort consisted of a minimum of 3 patients (completing at least cycle 1 of the background chemotherapy - for safety evaluation needed for escalation to the next dose level).

| **Cohort** | **1** | **2** | **3** | **4** |
|---|---|---|---|---|
| **Number of patients** | 3 | 3 | 4** | 6** |
| **Amphinex dose* (mg/kg)** | 0,06 | 0,06 | 0,12 | 0,25 |
| **Light dose (J/cm)** | | | | |
| (i.e. J per cm active diffuser length of the optical fibre) | 15 | 30 | 30 | 30 |

| | | | | |
|---|---|---|---|---|
| * Dose of TPCS₂ₐ.2MEA ** One patient each in Cohort 3 and 4 were withdrawn - did not receive/complete Cycle 1 of the background treatment (a requirement for complete safety evaluation of a cohort) | | | | |

### Results - Safety:

No Dose Limiting Toxicity (DLT) was observed and there was no apparent increase in adverse reactions with increasing doses. The most common Adverse Events were: mild photosensitivity reactions, abdominal pain, and cholangitis.

### Results - Efficacy:

Patients were examined by CT or MR at baseline, and approximately 3 and 6 months after the start of cycle 1 of the standard chemotherapy treatment. All images were evaluated separately by two experts in RECIST (response evaluation criteria in solid tumour) and bile duct cancer. Evaluation was conducted at the local level by experts at the hospital treating the patient (local read) for cohorts 1-4 or by the two central independent radiological experts (central read) for cohorts 3 and 4. For the central read, in the case of dispute between the readers, an adjudicator was used for evaluation.

The applied dose levels in cohorts 1 to 3 were below what was expected to be effective (Sultan et al., 2016, Lancet Oncology 17:1217-29).

6 months results from Cohorts 3 and 4 (7 evaluable of 9 patients): 2 with Progressive Disease (PD - >20% growth), 1 with Stable Disease (SD), 2 with Partial Response (PR - >30% shrinkage) and 2 with Complete Response (CR - no visible tumour) (via central read). The objective response rate (PR+CR) was more than 50 % (4/7 patients with hilar carcinoma, naive treatment), which is far above that expected with standard treatment (combination of gemcitabine and cisplatin (Valle et al, 2010, N. Engl. J. Med., 362:1273-81), 13 patients with hilar carcinoma, naive treatment).

Evaluation of the response at patient level from cohorts 3 and 4 (central read) is shown in Figure 7 compared to the standard chemotherapy treatment (ABC02). Evaluation of the response at target lesion level from cohorts 3 and 4, assessing **the sum of all** target (= measurable) lesions identified by the two central independent radiological experts (central read) is shown in Figure 8. Evaluation of the response at target lesion level from cohorts 3 and 4, assessing **the anticipated treated** target (= measurable) lesions identified by the two central independent radiological experts (central read) is shown in Figure 9.

The results show more than 50% of patients in cohorts 3 and 4 (treated with TPSC₂ₐ at 0.12 or 0.25 mg/kg and exposed to a light dose of 30 J/cm of the fiber) exhibited a partial or complete response to treatment at 6 months whereas treatment using standard chemotherapy resulted in partial treatment of less than 10% of patients (1/13) (Figure 7). Furthermore, almost 90% of selected target lesions exhibited shrinkage with more than 60% being undetectable at 6 months (Figure 8). Similar results were observed when examining anticipated treated target lesions (Figure 9). It will be noted from Figure 9 that only 14 tumours were anticipated to be treated (i.e. in the illumination area). However, as noted in Figure 8 when considering all tumours, including those not in the irradiation area, of 19 tumours 17 showed a response. This indicates that at least 3 of the tumours outside the illumination area responded to treatment suggesting that the effect extends beyond the illumination area. It is considered possible that this may result from the release of molecules from the irradiated tumours that may stimulate a local immune response which targets tumours outside the irradiation site. These results are of great significance offering treatment of cholangiocarcinomas to patients where previously no effective treatment was available.

The effect on overall target tumour size for each radiologically evaluable patient from all cohorts is shown in Figure 10. For cohorts 1 and 2 local reading results are shown. For cohorts 3 and 4 central reading results are shown. Patients 4 and 11 were excluded as they were not evaluable by radiology, i.e. their lesions could not be measured to allow a tumour reduction calculation. Figure 10 shows that approximately 90% of all patients showed a reduction in tumour size.

An overview of the patients examined, their RECIST results and their overall survival (OS) is shown in Table 2 below. The two columns for RECIST at 6 months shows the local read (left hand column) and central read (right hand column). Four patients remain alive (patients 6, 12, 14 and 16). The average survival to date is 16 months (see final column) and the median survival is 14.4 months (data not shown). In light of the four patients that remain alive it is expected that the average survival will increase beyond 16 months for the patients in the study.

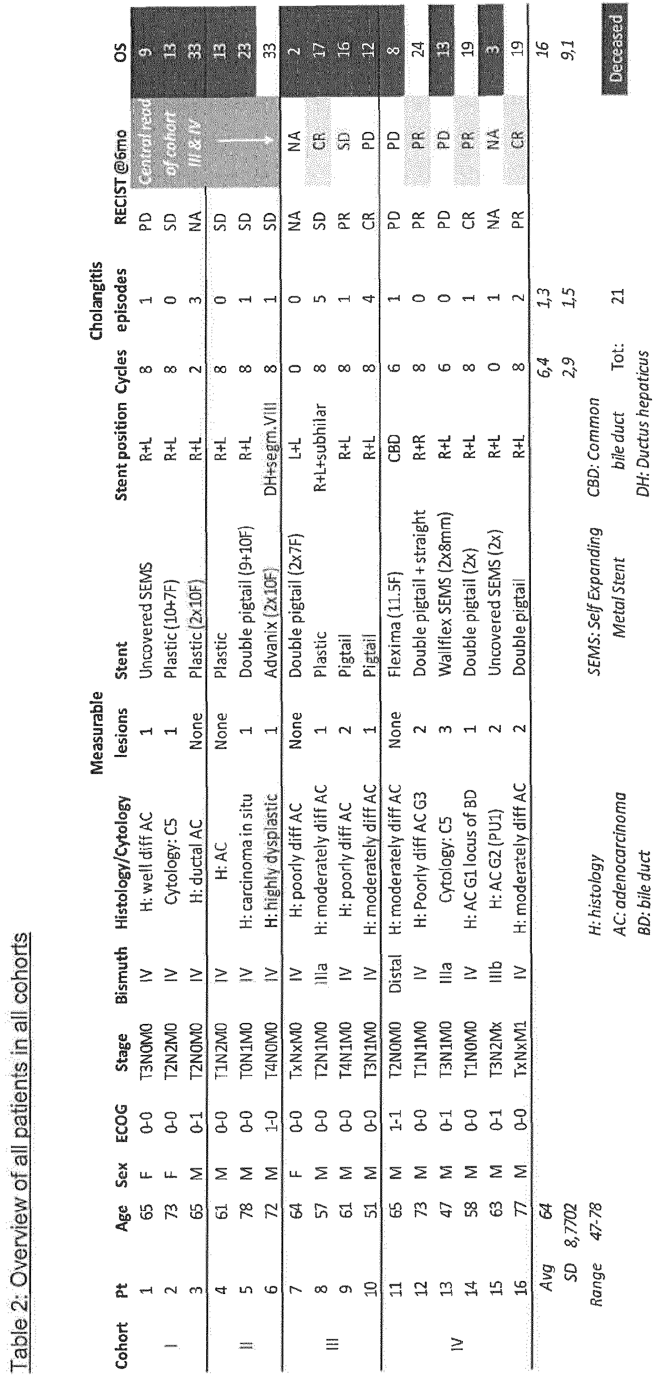

### EXAMPLE 4: In vivo (humans) evaluation of PCI with gemcitabine in treatment of cholangiocarcinoma (alternative treatment protocol)

The study is to assess the efficacy of Amphinex®-induced photochemical internalisation (PCI) of gemcitabine followed by gemcitabine/cisplatin chemotherapy in patients with advanced inoperable cholangiocarcinomas using a second round of PCI treatment.

### Study population:

Male and female patients with histologically or cytologically diagnosed inoperable, locally advanced or metastatic, cholangiocarcinoma who were chemo-naïve.

### Drug:

Amphinex 30 mg/ml, as in Example 3.

### Treatment:

Treatment is conducted as in Example 3 but with the following timing:

### a) First PCI treatment

Days 0 and 4: Treatment with Amphinex® (fimaporfin) and Gemcitabine, followed by irradiation (2-4 hours after completion of the gemcitabine administration) as described in Example 3 to achieve a dose of 30 J/cm.

### b) Standard systemic chemotherapy

Commencing between 7 or 21 days after the laser light application, patients receive 4 cycles of standard systemic chemotherapy: cisplatin (25 mg/m²) and gemcitabine (1000 mg/m²), given on Day 1 and on Day 8 of each 21-day cycle, as described in Example 3.

### c) Second PCI treatment

On day 18 of the 4^{th} cycle and day 1 of the 5^{th} cycle of standard systemic chemotherapy, PCI treatment is repeated as at days 0 and 4, above. Initiation of the second PCI treatment (= day 18 of cycle 4), and the start of subsequent systemic chemotherapy, may be postponed by up to 4 weeks, depending on the health condition of the patient.

### d) Standard systemic chemotherapy

Cycle 5 commences on the day after day 21 of cycle 4 (unless delayed due to the patient's health, as above). Systemic chemotherapy is conducted as set out in b) but on day 8 only in cycle 5. Three additional cycles are conducted as set out in b) (i.e. treatment on days 8 and 21).

Safety and efficacy is assessed as described in Example 3.

## Claims

1. Gemcitabine and TPCS₂ₐ and optionally another cytotoxic agent, preferably cisplatin, for use in treating a cholangiocarcinoma in a human patient, wherein
i) said TPCS₂ₐ is to be systemically administered to said patient to a dose of 0.05 to 0.5 mg/kg; and
ii) after 3-5 days, said gemcitabine is to be systemically administered to said patient to a dose of 500-1500 mg/m² and said cholangiocarcinoma is to be irradiated with light with a wavelength of 640-665nm using an optical fiber placed within 3cm of said cholangiocarcinoma to provide a light dose of 10 to 60 J/cm; and optionally
iii) after 1-40 days (preferably after 7-21 days) gemcitabine and/or another cytotoxic agent, preferably cisplatin, is to be systemically administered to said patient.

2. Gemcitabine and TPCS₂ₐ and optionally another cytotoxic agent for the use as claimed in claim 1 wherein said TPCS₂ₐ is to be administered to a dose of 0.1 to 0.5 mg/kg or 0.05 to 0.3 mg/kg, preferably 0.2 to 0.3 mg/kg.

3. Gemcitabine and TPCS₂ₐ and optionally another cytotoxic agent for the use as claimed in claim 1 or 2 wherein said gemcitabine in step ii) is to be administered to a dose of 900-1100 mg/m².

4. Gemcitabine and TPCS₂ₐ and optionally another cytotoxic agent for the use as claimed in any one of claims 1 to 3 wherein
i) said irradiation is to be performed within 4 hours of the start of gemcitabine administration; and/or
ii) the wavelength of said light is 652nm.

5. Gemcitabine and TPCS₂ₐ and optionally another cytotoxic agent for the use as claimed in any one of claims 1 to 4 wherein said light dose is 15 to 60 J/cm or 10 to 45 J/cm, preferably 25 to 35 J/cm.

6. Gemcitabine and TPCS₂ₐ and optionally another cytotoxic agent for the use as claimed in any one of claims 1 to 5 wherein in step iii) both gemcitabine and another cytotoxic agent, preferably cisplatin, are to be administered.

7. Gemcitabine and TPCS₂ₐ and optionally another cytotoxic agent for the use as claimed in any one of claims 1 to 6 wherein in step iii) the gemcitabine is to be administered to a dose of 500-1500 mg/m², preferably 900-1100 mg/m².

8. Gemcitabine and TPCS₂ₐ and optionally another cytotoxic agent for the use as claimed in any one of claims 1 to 7 wherein in step iii) the cytotoxic agent is cisplatin which is to be administered to a dose of 10-50 mg/m², preferably 20-30 mg/m².

9. Gemcitabine and TPCS₂ₐ and optionally another cytotoxic agent for the use as claimed in any one of claims 1 to 8 wherein in step iii) said gemcitabine and/or another cytotoxic agent, preferably cisplatin, is to be administered more than once.

10. Gemcitabine and TPCS₂ₐ and optionally another cytotoxic agent for the use as claimed in claim 9 wherein said gemcitabine and/or another cytotoxic agent, preferably cisplatin, is to be administered more than once (preferably twice) in a 14-30 day cycle that follows 1-40 days after steps i) and ii).

11. Gemcitabine and TPCS₂ₐ and optionally another cytotoxic agent for the use as claimed in claim 8 or 9 wherein said gemcitabine and/or another cytotoxic agent, preferably cisplatin, is to be administered once or more (preferably twice) in at least 3, preferably at least 5, 14-30 day cycles, wherein said first 14-30 day cycle follows 1-40 days after steps i) and ii).

12. Gemcitabine and TPCS₂ₐ and optionally another cytotoxic agent for the use as claimed in any one of claims 1 to 11 wherein steps (i), (ii) and (iii) are to be performed at least twice, wherein preferably in each round of steps (i), (ii) and (iii), step (iii) is to be performed at least twice.

13. Gemcitabine and TPCS₂ₐ and optionally another cytotoxic agent for the use as claimed in any one of claims 1 to 12 wherein the systemic administration of one or more of TPCS₂ₐ, gemcitabine and another cytotoxic agent, preferably cisplatin, is to be intravenous.

14. Gemcitabine and TPCS₂ₐ and optionally another cytotoxic agent for the use as claimed in any one of claims 1 to 7 and 9 to 13 wherein cisplatin is not to be used in said treatment.

15. Gemcitabine and TPCS₂ₐ and optionally another cytotoxic agent for the use as claimed in any one of claims 1 to 13 wherein said another cytotoxic agent is cisplatin.

16. A kit comprising gemcitabine and TPCS₂ₐ and optionally another cytotoxic agent as defined in any one of claims 1 to 3, 7 or 8 for simultaneous, separate or sequential use to treat a cholangiocarcinoma in a patient, wherein said use is as defined in any one of claims 1 to 15.

## Patentansprüche

1. Gemcitabin und TPCS₂ₐ und optional ein anderer zytotoxischer Wirkstoff, bevorzugt Cisplatin, zur Verwendung beim Behandeln eines Cholangiokarzinoms in einem menschlichen Patienten, wobei
i) das TPCS₂ₐ dem Patienten bei einer Dosis von 0,05 bis 0,5 mg/kg systemisch zu verabreichen ist; und
ii) nach 3-5 Tagen das Gemcitabin dem Patienten bei einer Dosis von 500-1500 mg/m² systemisch zu verabreichen ist und das Cholangiokarzinom mit Licht mit einer Wellenlänge von 640-665 nm unter Verwendung einer optischen Faser zu bestrahlen ist, die innerhalb von 3 cm vom Cholangiokarzinom platziert wird, um eine Lichtdosis von 10 bis 60 J/cm bereitzustellen; und optional
iii) nach 1-40 Tagen (bevorzugt nach 7-21 Tagen) Gemcitabin und/oder ein anderer zytotoxischer Wirkstoff, bevorzugt Cisplatin, dem Patienten systemisch zu verabreichen ist.

2. Gemcitabin und TPCS₂ₐ und optional ein anderer zytotoxischer Wirkstoff zur Verwendung nach Anspruch 1, wobei das TPCS₂ₐ bei einer Dosis von 0,1 bis 0,5 mg/kg oder 0,05 bis 0,3 mg/kg, bevorzugt 0,2 bis 0,3 mg/kg, zu verabreichen ist.

3. Gemcitabin und TPCS₂ₐ und optional ein anderer zytotoxischer Wirkstoff zur Verwendung nach Anspruch 1 oder 2, wobei das Gemcitabin in Schritt ii) bei einer Dosis von 900-1100 mg/m² zu verabreichen ist.

4. Gemcitabin und TPCS₂ₐ und optional ein anderer zytotoxischer Wirkstoff zur Verwendung nach einem der Ansprüche 1 bis 3, wobei
i) die Bestrahlung innerhalb von 4 Stunden nach dem Beginn einer Gemcitabin-Verabreichung durchzuführen ist; und/oder
ii) die Wellenlänge des Lichts 652 nm beträgt.

5. Gemcitabin und TPCS₂ₐ und optional ein anderer zytotoxischer Wirkstoff zur Verwendung nach einem der Ansprüche 1 bis 4, wobei die Lichtdosis 15 bis 60 J/cm oder 10 bis 45 J/cm, bevorzugt 25 bis 35 J/cm, beträgt.

6. Gemcitabin und TPCS₂ₐ und optional ein anderer zytotoxischer Wirkstoff zur Verwendung nach einem der Ansprüche 1 bis 5, wobei im Schritt iii) sowohl Gemcitabin als auch ein anderer zytotoxischer Wirkstoff, bevorzugt Cisplatin, zu verabreichen sind.

7. Gemcitabin und TPCS₂ₐ und optional ein anderer zytotoxischer Wirkstoff zur Verwendung nach einem der Ansprüche 1 bis 6, wobei im Schritt iii) das Gemcitabin bei einer Dosis von 500-1500 mg/m², bevorzugt 900-1100 mg/m², zu verabreichen ist.

8. Gemcitabin und TPCS₂ₐ und optional ein anderer zytotoxischer Wirkstoff zur Verwendung nach einem der Ansprüche 1 bis 7, wobei im Schritt iii) der zytotoxische Wirkstoff Cisplatin ist, der bei einer Dosis von 10-50 mg/m², bevorzugt 20-30 mg/m², zu verabreichen ist.

9. Gemcitabin und TPCS₂ₐ und optional ein anderer zytotoxischer Wirkstoff zur Verwendung nach einem der Ansprüche 1 bis 8, wobei im Schritt iii) das Gemcitabin und/oder ein anderer zytotoxischer Wirkstoff, bevorzugt Cisplatin, mehr als einmal zu verabreichen ist.

10. Gemcitabin und TPCS₂ₐ und optional ein anderer zytotoxischer Wirkstoff zur Verwendung nach Anspruch 9, wobei das Gemcitabin und/oder ein anderer zytotoxischer Wirkstoff, bevorzugt Cisplatin, mehr als einmal (bevorzugt zweimal) in einem 14-30-Tage-Zyklus zu verabreichen ist, der 1-40 Tage nach den Schritten i) und ii) folgt.

11. Gemcitabin und TPCS₂ₐ und optional ein anderer zytotoxischer Wirkstoff zur Verwendung nach Anspruch 8 oder 9, wobei das Gemcitabin und/oder ein anderer zytotoxischer Wirkstoff, bevorzugt Cisplatin, einmal oder mehr (bevorzugt zweimal) in mindestens 3, bevorzugt mindestens 5, 14-30-Tage-Zyklen zu verabreichen ist, wobei der erste 14-30-Tage-Zyklus 1-40 Tage nach den Schritten i) und ii) folgt.

12. Gemcitabin und TPCS₂ₐ und optional ein anderer zytotoxischer Wirkstoff zur Verwendung nach einem der Ansprüche 1 bis 11, wobei die Schritte (i), (ii) und (iii) mindestens zweimal durchzuführen sind, wobei bevorzugt in jeder Runde der Schritte (i), (ii) und (iii) der Schritt (iii) mindestens zweimal durchzuführen ist.

13. Gemcitabin und TPCS₂ₐ und optional ein anderer zytotoxischer Wirkstoff zur Verwendung nach einem der Ansprüche 1 bis 12, wobei die systemische Verabreichung von einem oder mehreren von TPCS₂ₐ, Gemcitabin und einem anderen zytotoxischen Wirkstoff, bevorzugt Cisplatin, intravenös zu erfolgen hat.

14. Gemcitabin und TPCS₂ₐ und optional ein anderer zytotoxischer Wirkstoff zur Verwendung nach einem der Ansprüche 1 bis 7 und 9 bis 13, wobei Cisplatin nicht in der Behandlung zu verwenden ist.

15. Gemcitabin und TPCS₂ₐ und optional ein anderer zytotoxischer Wirkstoff zur Verwendung nach einem der Ansprüche 1 bis 13, wobei ein anderer zytotoxischer Wirkstoff Cisplatin ist.

16. Kit, umfassend Gemcitabin und TPCS₂ₐ und optional einen anderen zytotoxischen Wirkstoff wie in einem der Ansprüche 1 bis 3, 7 oder 8 definiert, zur gleichzeitigen, separaten oder aufeinanderfolgenden Verwendung zum Behandeln eines Cholangiokarzinoms in einem Patienten, wobei die Verwendung wie in einem der Ansprüche 1 bis 15 definiert erfolgt.

## Revendications

1. Gemcitabine et TPCS₂ₐ et facultativement un autre agent cytotoxique, de préférence le cisplatine, pour leur utilisation dans le traitement d'un cholangiocarcinome chez un patient humain, dans lesquels
i) ledit TPCS₂ₐ doit être administré de manière systémique audit patient à une dose de 0,05 à 0,5 mg/kg ; et
ii) après 3 à 5 jours, ladite gemcitabine doit être administrée de manière systémique audit patient à une dose de 500 à 1500 mg/m² et ledit cholangiocarcinome doit être exposé à une lumière ayant une longueur d'onde de 640 à 665 nm en utilisant une fibre optique placée au plus à 3 cm dudit cholangiocarcinome pour fournir une dose de lumière de 10 à 60 J/cm ; et facultativement
iii) après 1 à 40 jours (de préférence après 7 à 21 jours) la gemcitabine et/ou un autre agent cytotoxique, de préférence le cisplatine, doivent être administrés de manière systémique audit patient.

2. Gemcitabine et TPCS₂ₐ et facultativement un autre agent cytotoxique pour leur utilisation selon la revendication 1 dans lesquels ledit TPCS₂ₐ doit être administré à une dose de 0,1 à 0,5 mg/kg ou 0,05 à 0,3 mg/kg de préférence de 0,2 à 0,3 mg/kg.

3. Gemcitabine et TPCS₂ₐ et facultativement un autre agent cytotoxique pour leur utilisation selon la revendication 1 ou 2 dans lesquels ladite gemcitabine à l'étape ii) doit être administrée à une dose de 900 à 1100 mg/m².

4. Gemcitabine et TPCS₂ₐ et facultativement un autre agent cytotoxique pour leur utilisation selon l'une quelconque des revendications 1 à 3 dans lesquels
i) ladite exposition doit être réalisée dans les 4 heures qui suivent le début de l'administration de gemcitabine ; et/ou
ii) la longueur d'onde de ladite lumière est de 652 nm.

5. Gemcitabine et TPCS₂ₐ et facultativement un autre agent cytotoxique pour leur utilisation selon l'une quelconque des revendications 1 à 4 dans lesquels ladite dose de lumière est de 15 à 60 J/cm ou de 10 à 45 J/cm, de préférence de 25 à 35 J/cm.

6. Gemcitabine et TPCS₂ₐ et facultativement un autre agent cytotoxique pour leur utilisation selon l'une quelconque des revendications 1 à 5 dans lesquels à l'étape iii) à la fois la gemcitabine et un autre agent cytotoxique, de préférence le cisplatine, doivent être administrés.

7. Gemcitabine et TPCS₂ₐ et facultativement un autre agent cytotoxique pour leur utilisation selon l'une quelconque des revendications 1 à 6 dans lesquels à l'étape iii) la gemcitabine doit être administrée à une dose de 500 à 1500 mg/m², de préférence de 900 à 1100 mg/m².

8. Gemcitabine et TPCS₂ₐ et facultativement un autre agent cytotoxique pour leur utilisation selon l'une quelconque des revendications 1 à 7 dans lesquels à l'étape iii) l'agent cytotoxique est le cisplatine qui doit être administré à une dose de 10 à 50 mg/m², de préférence de 20 à 30 mg/m².

9. Gemcitabine et TPCS₂ₐ et facultativement un autre agent cytotoxique pour leur utilisation selon l'une quelconque des revendications 1 à 8 dans lesquels à l'étape iii) ladite gemcitabine et/ou un autre agent cytotoxique, de préférence le cisplatine, doivent être administrés plus d'une fois.

10. Gemcitabine et TPCS₂ₐ et facultativement un autre agent cytotoxique pour leur utilisation selon la revendication 9 dans lesquels ladite gemcitabine et/ou un autre agent cytotoxique, de préférence le cisplatine, doivent être administrés plus d'une fois (de préférence deux fois) en un cycle de 14 à 30 jours qui suit les 1 à 40 jours après les étapes i) et ii).

11. Gemcitabine et TPCS₂ₐ et facultativement un autre agent cytotoxique pour leur utilisation selon la revendication 8 ou 9 dans lesquels ladite gemcitabine et/ou un autre agent cytotoxique, de préférence le cisplatine, doivent être administrés une fois ou plus (de préférence deux fois) en au moins 3, de préférence au moins 5, cycles de 14 à 30 jours, dans lesquels ledit premier cycle de 14 à 30 jours suit les 1 à 40 jours après les étapes i) et ii).

12. Gemcitabine et TPCS₂ₐ et facultativement un autre agent cytotoxique pour leur utilisation selon l'une quelconque des revendications 1 à 11 dans lesquels les étapes (i), (ii) et (iii) doivent être réalisées au moins deux fois, dans lesquels de préférence à chaque passage des étapes (i), (ii) et (iii), l'étape (iii) doit être réalisée au moins deux fois.

13. Gemcitabine et TPCS₂ₐ et facultativement un autre agent cytotoxique pour leur utilisation selon l'une quelconque des revendications 1 à 12 dans lesquels l'administration systémique d'un ou de plusieurs du TPCS₂ₐ, de la gemcitabine et d'un autre agent cytotoxique, de préférence le cisplatine, doit être intraveineuse.

14. Gemcitabine et TPCS₂ₐ et facultativement un autre agent cytotoxique pour leur utilisation selon l'une quelconque des revendications 1 à 7 et 9 à 13 dans lesquels le cisplatine ne doit pas être utilisé dans ledit traitement.

15. Gemcitabine et TPCS₂ₐ et facultativement un autre agent cytotoxique pour leur utilisation selon l'une quelconque des revendications 1 à 13 dans lesquels ledit autre agent cytotoxique est le cisplatine.

16. Kit comprenant la gemcitabine et le TPCS₂ₐ et facultativement un autre agent cytotoxique selon l'une quelconque des revendications 1 à 3, 7 ou 8 pour une utilisation simultanée, séparée ou séquentielle pour le traitement d'un cholangiocarcinome chez un patient, dans lequel ladite utilisation est telle que définie dans l'une quelconque des revendications 1 à 15.
